# EUROPEAN PATENT APPLICATION

(11) **EP 4 411 359 A1**
(43) Date of publication of application: **07.08.2024**
(21) Application number: 24150952.0
(22) Date of filing: 09.01.2024
(51) Int. Cl.: G01N 27/12, G01N 27/02

(54) **SYSTEMS, APPARATUSES, AND METHODS FOR DETECTING A VAPOR**

(30) Priority: 03.02.2023 US 202318164314
(71) Applicant: Honeywell International Inc., Charlotte, NC 28202 (US)
(72) Inventor: CHAPPLES, John, Charlotte, 28202 (US); PRATT, Keith Francis Edwin, Charlotte, 28202 (US); FOLEY, Thomas, Charlotte, 28202 (US)
(74) Representative: Haseltine Lake Kempner LLP

(57) **Abstract**

Systems, apparatus, and methods for detecting a vapor are provided herein. The system for detecting a vapor may include a battery and a sensor disposed proximate the battery. The sensor of the system for detecting a vapor may further include a substrate. The sensor of the system for detecting a vapor may further include a pair of electrodes disposed on the substrate. The sensor of the system for detecting a vapor may further include a polymer support disposed on the substrate such that the polymer support is in contact with the pair of electrodes. In this regard, the polymer support may include an ionic salt. The polymer support may be configured to absorb at least some of the vapor and, when the polymer support absorbs at least some of the vapor, a conductivity of the polymer support may increase.

## Description

### TECHNOLOGICAL FIELD

Embodiments of the present disclosure relate generally to systems, apparatuses, and methods for detecting a vapor.

### BACKGROUND

Applicant has identified many technical challenges and difficulties associated with systems, apparatuses, and methods for detecting a vapor. Through applied effort, ingenuity, and innovation, Applicant has solved problems related to systems, apparatuses, and methods for detecting a vapor by developing solutions embodied in the present disclosure, which are described in detail below.

### BRIEF SUMMARY

Various embodiments described herein relate to systems, apparatuses, and methods for detecting a vapor.

In accordance with one aspect of the disclosure, a sensor for detecting a vapor is provided. In some embodiments, the sensor for detecting a vapor may include a substrate. In some embodiments, the sensor for detecting a vapor may include a pair of electrodes disposed on the substrate. In some embodiments, the sensor for detecting a vapor may include a polymer support disposed on the substrate such that the polymer support is in contact with the pair of electrodes. In some embodiments, the polymer support comprises an ionic salt. In some embodiments, the polymer support is configured to absorb at least some of the vapor. In some embodiments, when the polymer support absorbs at least some of the vapor, a conductivity of the polymer support increases.

In some embodiments, the polymer support absorbing at least some of the vapor causes the ionic salt to solvate.

In some embodiments, the ionic salt comprises one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide.

In some embodiments, the polymer support comprises one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI).

In some embodiments, the vapor comprises one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

In some embodiments, the sensor for detecting a vapor may include a pair of contact pads disposed on the substrate, each of the pair of contact pads in communication with one of the pair of electrodes.

In accordance with another aspect of the disclosure, a system for detecting a vapor is provided. In some embodiments, the system for detecting a vapor may include a battery. In some embodiments, the system for detecting a vapor may include a sensor disposed proximate the battery. In some embodiments, the sensor may include a substrate. In some embodiments, the sensor may include a pair of electrodes disposed on the substrate. In some embodiments, the sensor may include a polymer support disposed on the substrate such that the polymer support is in contact with the pair of electrodes. In some embodiments, the polymer support comprises an ionic salt. In some embodiments, the polymer support is configured to absorb at least some of the vapor. In some embodiments, when the polymer support absorbs at least some of the vapor, a conductivity of the polymer support increases.

In some embodiments, the polymer support absorbing at least some of the vapor causes the ionic salt to solvate.

In some embodiments, the ionic salt comprises one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide.

In some embodiments, the polymer support comprises one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI).

In some embodiments, the vapor comprises one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

In some embodiments, the battery releases the vapor when a temperature of the battery is greater than a temperature threshold.

In some embodiments, the battery releases the vapor due to a fault associated with the battery.

In some embodiments, the system may include a computing device in communication with the sensor and configured to measure an impedance of the polymer support or a phase angle associated with the polymer support.

In accordance with another aspect of the disclosure, a method for detecting a vapor is provided. In some embodiments, the method includes measuring an impedance of a polymer support. In some embodiments, the polymer support is disposed on a substrate such that the polymer support is in contact with a pair of electrodes disposed on the substrate. In some embodiments, the polymer support comprises an ionic salt. In some embodiments, the polymer support is configured to absorb at least some of the vapor. In some embodiments, when the polymer support absorbs at least some of the vapor, a conductivity of the polymer support increases.

In some embodiments, the ionic salt comprises one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide.

In some embodiments, the polymer support comprises one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI).

In some embodiments, the vapor comprises one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

In some embodiments, the vapor is detected when the impedance of the polymer support is below an impedance threshold.

In some embodiments, the method may optionally include measuring a phase angle associated with the polymer support.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the disclosure in any way. It will be appreciated that the scope of the present disclosure encompasses many potential embodiments in addition to those here summarized, some of which will be further described below.

### BRIEF DESCRIPTION OF THE DRAWINGS

Reference will now be made to the accompanying drawings. The components illustrated in the figures may or may not be present in certain embodiments described herein. Some embodiments may include fewer (or more) components than those shown in the figures in accordance with an example embodiment of the present disclosure.
FIG. 1 illustrates an overview of an example system for detecting a vapor in accordance with one or more embodiments of the present disclosure;
FIG. 2 illustrates a cross-sectional view of an example sensor in accordance with one or more embodiments of the present disclosure;
FIG. 3 illustrates an example front view of an example polymer support in accordance with one or more embodiments of the present disclosure;
FIG. 4 illustrates an example front view of an example polymer support in accordance with one or more embodiments of the present disclosure;
FIG. 5 illustrates an overview of example batteries of the example system for detecting a vapor in accordance with one or more embodiments of the present disclosure;
FIG. 6 illustrates an overview of an example battery pack of the example system for detecting a vapor in accordance with one or more embodiments of the present disclosure;
FIG. 7 illustrates an overview of an example battery pack of the example system for detecting a vapor in accordance with one or more embodiments of the present disclosure;
FIG. 8 illustrates an overview of an example battery pack of the example system for detecting a vapor in accordance with one or more embodiments of the present disclosure;
FIG. 9 illustrates an example impedance graph in accordance with one or more embodiments of the present disclosure;
FIG. 10 illustrates an example graph depicting a phase angle of an impedance in accordance with one or more embodiments of the present disclosure;
FIG. 11 illustrates an overview of the example system for detecting a vapor implemented in an electric vehicle in accordance with one or more embodiments of the present disclosure;
FIG. 12 illustrates an example user interface of a computing device in accordance with one or more embodiments of the present disclosure;
FIG. 13 illustrates a flowchart of an example method for detecting a vapor in accordance with one or more embodiments of the present disclosure; and
FIG. 14 illustrates a block diagram of an example computer processing device in accordance with one or more embodiments of the present disclosure.

### DETAILED DESCRIPTION

Example embodiments will be described more fully hereinafter with reference to the accompanying drawings, in which some, but not all embodiments of disclosure are shown. Indeed, embodiments of the disclosure may be embodied in many different forms and should not be construed as limited to the embodiments set forth herein; rather, these embodiments are provided so that this disclosure will satisfy applicable legal requirements. Like numbers refer to like elements throughout.

### Overview

Example embodiments disclosed herein address technical problems associated with systems, apparatuses, and methods for detecting a vapor. As would be understood by one skilled in the field to which this disclosure pertains, there are numerous example scenarios in which a user may use systems, apparatuses, and methods for detecting a vapor.

In many applications, it is often necessary to detect a vapor. For example, it may be necessary to detect a vapor associated with a battery (e.g., an electrolyte vapor that may be released from a battery) to detect if the battery is suffering from a fault and/or is overheating (e.g., due to a thermal runaway). In this regard, due to a variety of catalysts, such as poor design, overcharging, over-discharging, physical damage, and/or exposure to extreme temperatures (e.g., high temperatures cause the battery to overheat), batteries are susceptible to malfunctioning and/or having a thermal runaway. In a battery fault and/or a thermal runaway, the temperature of a battery may increase to a point that causes a chemical reaction to occur inside the battery, which further increases the temperature of the battery and causes more chemical reactions to occur. A battery fault and/or a thermal runaway may cause the battery to suffer a catastrophic failure, such as catching on fire, which may destroy the battery and also cause damage to nearby objects and/or individuals.

The resulting catastrophic failure of a battery, due to battery faults and/or thermal runaway, is particularly problematic in devices requiring large amounts of power, such as electric vehicles, because these devices may include hundreds of batteries (e.g., the hundreds of batteries may be organized into multiple battery packs with each battery pack including several batteries). As a result, if one battery suffers a catastrophic failure, this may cause other batteries in the battery pack and/or batteries in other battery packs to suffer a catastrophic failure (e.g., the catastrophic failure of one battery in an electric vehicle may cause all of the batteries in an electric vehicle to catch on fire and suffer a catastrophic failure). Thus, resulting in the destruction of the electric vehicle, nearby objects (e.g., a garage the electric vehicle is parked in), and/or harm to nearby individuals.

In order to mitigate the risk of battery faults and/or thermal runaway, many batteries include a vent through which heat and vapors (e.g., vapors generated from chemical reactions in the battery) may be released. For example, vapors may be released through a battery vent. However, although a battery vent may allow some heat and vapors to escape from the battery, in many cases the vent alone is not capable of preventing a battery having a battery fault and/or in thermal runaway from suffering a catastrophic failure. As such, corrective action (e.g., stopping the charging of the battery) must be made by a user associated with the battery and/or other systems associated with the battery (e.g., a computing device associated with the battery). Accordingly, detecting a vapor may enable a user associated with the battery and/or other systems associated with the battery to take corrective action to remedy a battery having a fault and/or in thermal runaway before the battery suffers a catastrophic failure.

Example solutions for detecting a vapor include sensors not specifically designed to detect vapors, such as, for example, a metal oxide sensor. However, metal oxide sensors have several drawbacks. For example, metal oxide sensors are generally non-specific sensors and, as a result, respond to any vapor that can modify the surface oxidation of the metal oxide sensor (e.g., including vapors that are not desired to be detected) and/or have low sensitivity (e.g., can only detect vapors when there is a high concentration of the vapor near the sensor). As another example, metal oxide sensors use a moving average baseline to detect vapors and, as a result, metal oxide sensors are unable to detect the slow buildup of a vapor. As another example, metal oxide sensors are prone to baseline drift over time which decreases the accuracy of the metal oxide sensors over time. As another example, metal oxide sensors may be large in size and, as a result may be difficult to place near batteries (e.g., it may be difficult to place metal oxide sensors near a battery when the battery is in a confined space, such as in an electric vehicle). As another example, metal oxide sensors may be expensive to produce. As another example, metal oxide sensors may consume a large amount of power when operating (e.g., more than 10 milliwatts (mW) because metal oxide sensors typically have to be heated to high temperatures (e.g., greater than 200°C) to operate) and, as a result, it may be difficult to provide enough power such that a desired number of metal oxide sensors can be used in an application having many batteries (e.g., in an application having hundreds of batteries, it may be desirable to use multiple metal oxide sensors, but, due to the power consumption of metal oxide sensors, it may only be possible to provide enough power to use a less than desired number of metal oxide sensors).

Accordingly, systems that use metal oxide sensors to detect a vapor often have high rates of false alarms (e.g., due to metal oxide sensors being non-specific sensors), become inaccurate over time (e.g., due to metal oxide sensors being prone to baseline drift), be unable to detect the slow buildup of a vapor (e.g., due to metal oxide sensors using a moving average baseline to detect vapors), and may be difficult and/or expensive to implement in many applications (e.g., due to metal oxide sensors being large in size, being expensive to produce, and consuming large amounts of power when operating). These example drawbacks of metal oxide sensors decrease the usefulness of metal oxide sensors. Accordingly, there is a need for systems, apparatuses, and methods, that are capable of accurately and efficiently detecting a vapor, such as systems, apparatuses, and methods, that are capable of accurately and efficiently detecting an electrolyte vapor released from a battery
Thus, to address these and/or other issues related to detecting a vapor, example systems, apparatuses, and methods are disclosed herein. For example, an embodiment in this disclosure, described in greater detail below, includes a sensor that is able to detect a vapor. In some examples, the sensor may include a substrate, a pair of electrodes disposed on the substrate, and a polymer support disposed on the substrate such that the polymer support is in contact with the pair of electrodes. In some examples, the polymer support may include an ionic salt. In some examples, the polymer support may be configured to absorb at least some of the vapor and, when the polymer support absorbs at least some of the vapor, a conductivity of the polymer support may increase. In some examples, the impedance of the polymer support and/or a phase angle associated with the polymer support may be measured and, if the impedance of the polymer support is below an impedance threshold (e.g., because the conductivity has increased) and/or the phase angle associated with the polymer support exceeds a phase angle threshold (e.g., because the conductivity has increased), a vapor is detected. Accordingly, in some examples, the sensor may be able to accurately detect (e.g., without a high risk of false alarms) a vapor released from a battery having a battery fault and/or in thermal runaway in a variety of applications and circumstances. Thus, enabling a user associated with the battery and/or systems associated with the battery to take corrective action before the battery suffers a catastrophic failure.

### Example System for Detecting a Vapor

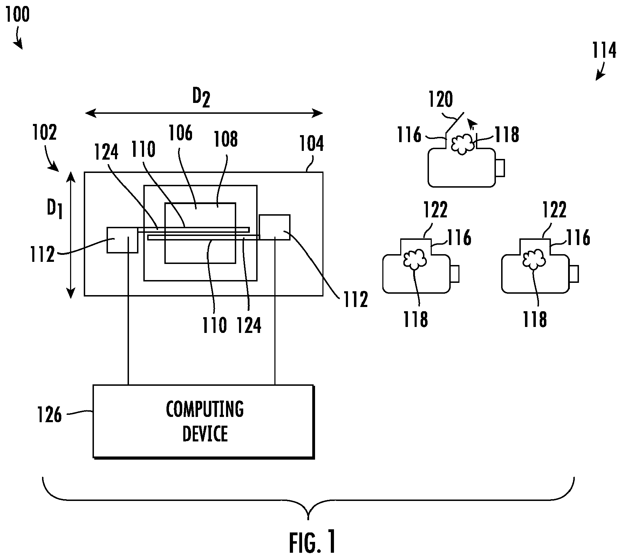

With reference to FIGS. 1-5 embodiments herein provide for an example system for detecting a vapor 100. In some embodiments, the system for detecting a vapor 100 may include a sensor 102. In some embodiments, the sensor 102 may be able to detect any vapor that may solvate an ionic salt. For example, the sensor 102 may be able to detect a vapor associated with a battery (e.g., an electrolyte vapor that may be released from a battery). In some embodiments, the sensor 102 may have dimensions D₁ and D₂. In some embodiments, D₁ may be less than 10mm and/or D₂ may be less than 10mm. For example, in some embodiments, D₁ may be approximately 1mm and/or D₂ may be approximately 1 mm.

In some embodiments, the sensor 102 may include a substrate 104. The substrate 104 may comprise one or more of silicon, silicon oxide, silicon nitride, borosilicate glass, quartz, silica, sapphire, alumina, or plastic. Said differently, the substrate 104 may be comprised of any material capable of ensuring that the sensor 102 may detect a vapor 118. In this regard, for example, the substrate 104 may be a printed circuit board.

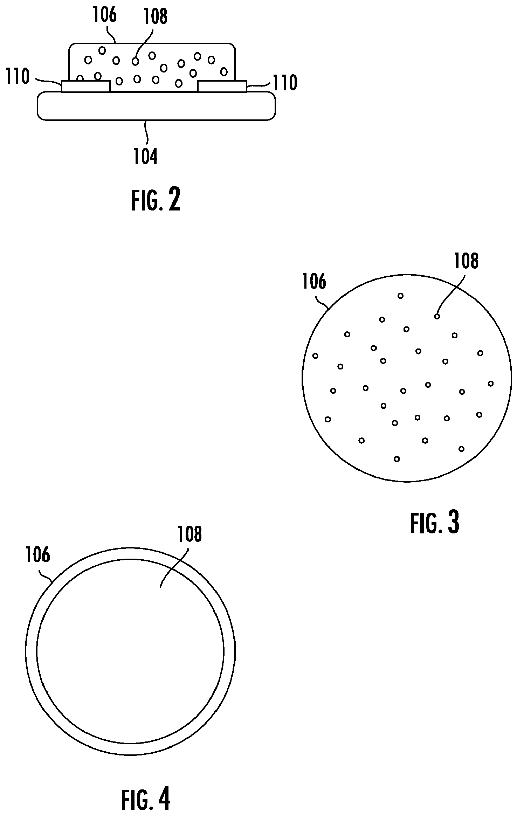

In some embodiments, the sensor 102 may include a pair of electrodes 110 disposed on the substrate 104. In some embodiments, the sensor 102 may include a polymer support 106 disposed on the substrate 104. The polymer support 106 may be disposed on the substrate 104 such that the polymer support 106 is in contact with each of the pair of electrodes 110. The pair of electrodes 110 may comprise one or more of copper, nickel, cobalt, tungsten, silicon carbide, palladium, platinum, gold, or transition metal alloys. For example, as depicted in FIGS. 1 and 2, each of the pair of electrodes 110 may be in contact with a portion of the polymer support 106. In this regard, for example, the polymer support 106 may be configured to provide a connection between the pair of electrodes 110 (e.g., the pair of electrodes 110 may not be in contact with each other). In some embodiments, the pair of electrodes 110 may be disposed on the substrate 104 in any configuration such that the impedance of the polymer support 106 and/or phase angle associated with the polymer support 106 may be measured. For example, each of the pair of electrodes 110 may be interdigitated electrodes. As another example, each of the pair of electrodes 110 may be disposed on the substrate 104 such that the pair of electrodes 110 form a spiral. In some embodiments, a distance between the pair of electrodes 110 may be approximately equal to a thickness of the polymer support 106.

In some embodiments, the polymer support 106 maybe any shape such that when disposed on the substrate 104, the polymer support 106 may be in contact with the pair of electrodes 110. For example, the polymer support 106 may be cylindrical, cubical, rectangular, and/or the like. In some embodiments, the polymer support 106 may be comprised of one or more thermoplastics (e.g., a non-reactive thermoplastic). For example, the polymer support 106 may comprise one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), Poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI).

In some embodiments, the polymer support 106 may include an ionic salt 108. In some embodiments, the ionic salt 108 may be dispersed throughout the polymer support 106. For example the ionic salt 108 may be dispersed in clumps throughout the polymer support 106. As another example, the ionic salt 108 may be dispersed throughout the polymer support 106 in a substantially uniform distribution. In some embodiments, the ionic salt 108, may be dispersed in clumps throughout the polymer support 106 and/or throughout the polymer support 106 in a substantially uniform distribution before the sensor 102 has detected a vapor 118.

In some embodiments, the ionic salt 108 may be comprised of one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide. In some embodiments, the ionic salt 108 may be chosen based on the ionic salt's 108 solubility in the vapor 118, the ionic salt's 108 ability to form an ionically conducting electrolyte after the ionic salt 108 has been exposed to the vapor 118, and/or the ionic salt's 108 compatibility with the polymer support 106.

In some embodiments, the polymer support 106 may be configured to absorb at least some of the vapor 118. For example, the polymer support 106 may absorb some of a vapor 118 when a vapor 118 is in the presence of the sensor 102. In some embodiments, absorbing at least some of a vapor 118 may cause the polymer support 106 to solvate. In this regard, for example, the polymer support 106 may become more flexible. In some embodiments, absorbing at least some of a vapor 118 may cause the ionic salt 108 to solvate. In this regard, such as depicted in FIG. 4 for example, the ionic salt 108 may dissolve into the polymer support 106 (e.g., the ionic salt 108 may dissociate into ions). In some embodiments, the polymer support 106 may absorb some of a vapor 118 (and solvate the polymer support 106 and/or the ionic salt 108) within one minute of the vapor 118 being in the presence of the sensor 102. In some embodiments, absorbing at least some of a vapor 118 and, as a result, causing the polymer support 106 and/or the ionic salt 108 to solvate, causes a conductivity of the polymer support 106 to increase. In this regard, for example, if the conductivity of the polymer support 106 increases, the impedance of the polymer support 106 may decrease. As another example, if the conductivity of the polymer support 106 increases, the phase angle associated with the polymer support 106 (e.g., the phase angle of the impedance of the polymer support 106) may shift (e.g., the phase angle associated with the polymer support 106 may shift such that the phase angle exceeds a phase angle threshold).

In some embodiments, the vapor 118 may be any vapor that may solvate the ionic salt 108. For example, as described above, the vapor 118 may be associated with one or more batteries 114. That is, in some embodiments, the vapor 118 may be released from one of the one or more batteries 114. In some embodiments, the vapor 118 may be comprised of one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

In some embodiments, the sensor 102 may include a pair of contact pads 112 disposed on the substrate 104. In some embodiments, each of the pair of contact pads 112 may be in communication (e.g., electrical communication) with one of the pair of electrodes 110. In some embodiments, each of the pair of contact pads 112 may be in communication (e.g., electrical communication) with one of the pair of electrodes 110 via an associated connection path 124. In some embodiments, each connection path 124 may comprise one or more electrical connection members, such as electrical wires, electrical leads, electrical traces, and/or the like.

In some embodiments, each of the pair of contact pads 112 may be configured to be connected to a computing device 126. In this regard, the sensor 102 may be configured to be connected to the computing device 126 via each of the pair of contact pads 112 such that the sensor 102 and the computing device 126 may be in communication (e.g., electrical communication). In some embodiments, the sensor 102 and the computing device 126 may be connected by one or more electrical connection members, such as electrical wires, electrical leads, electrical trades, and/or the like. Although the sensor 102 and the computing device 126 are depicted as separate components in FIG. 1, it would be understood by one skilled in the field to which this disclosure pertains that, in some embodiments, the sensor 102 and the computing device 126 may be combined into a single component. For example, the sensor 102 may integrated into the computing device 126 or the computing device 126 may be integrated into the sensor 102.

As described above, in some embodiments, the system for detecting a vapor 100 may include one or more batteries 114. The one or more batteries 114 may be any kind of battery used in a variety of applications (e.g., in electric vehicles). For example, the one or more batteries 114 may include lithium-ion batteries, lithium-polymer batteries, alkaline batteries, nickel metal hydride batteries, carbon zinc batteries, silver oxide batteries, zinc air batteries, single use batteries, rechargeable batteries, and/or the like. In some embodiments, the one or more batteries 114 may include more than one type of battery. For example, one of the one or more batteries 114 may be a lithium-ion battery and another of the one or more batteries 114 may be a lithium-polymer battery.

In some embodiments, each of the one or more batteries 114 may include a vent 116. In some embodiments, the vent 116 may be located at any location on each of the one or more batteries 114 (e.g., on the top of each of the one or more batteries 114). The vent 116 of each of the one or more batteries 114 may be configured to move between a closed position 122 and an open position 120. In some embodiments, the vent 116 of each of the one or more batteries 114 may be in the closed position 122 when the battery is operating normally (e.g., the battery is undamaged and/or operating at a normal temperature). In some embodiments, the vent 116 of each of the one or more batteries 114 may be in the open position 120 when the battery is not operating normally (e.g., the battery is damaged and/or not operating at a normal temperature). For example, the vent 116 of one of the one or more batteries 114 may be in the open position 120 when a temperature of the battery is above a temperature threshold. In some embodiments, the vent 116 of one of the one or more batteries 114 may be configured to move from the closed position 122 to the open position 120 when the temperature of the battery is above the temperature threshold. In some embodiments, the temperature threshold may be based on the size (e.g., the size of a battery cell in the one or more batteries 114), material (e.g., the material of a battery cell in the one or more batteries 114), and/or design (e.g., the design of a battery cell in the one or more batteries 114) of the one or more batteries 114. In some embodiments, the temperature threshold may be between approximately 130°C and 200°C.

In some embodiments, the vent 116 of one or more of the one or more batteries 114 may be configured to move from the closed position 122 to the open position 120 to release a vapor 118 from the battery and, in some embodiments, heat from the battery. In some embodiments, the vent 116 of one of the one or more batteries 114 may be configured to move from the closed position 122 to the open position 120 to release a vapor from the battery when the battery is overheating (e.g., going into thermal runaway) and/or due to a fault associated with the battery (e.g., a fault not associated with the battery overheating). For example, as the temperature of one of the one or more batteries 114 increases, a vapor 118 may build up inside the battery. In some embodiments, once a certain amount of the vapor 118 has built up inside one of the one or more batteries 114 (e.g., when the temperature of the battery is above the temperature threshold), the vent 116 may move from the closed position 122 to the open position 120 to release the vapor 118 and reduce the pressure inside the battery. In this regard, the release of the vapor 118 may indicate that the battery is not operating normally (e.g., the battery is overheating and going into thermal runaway).

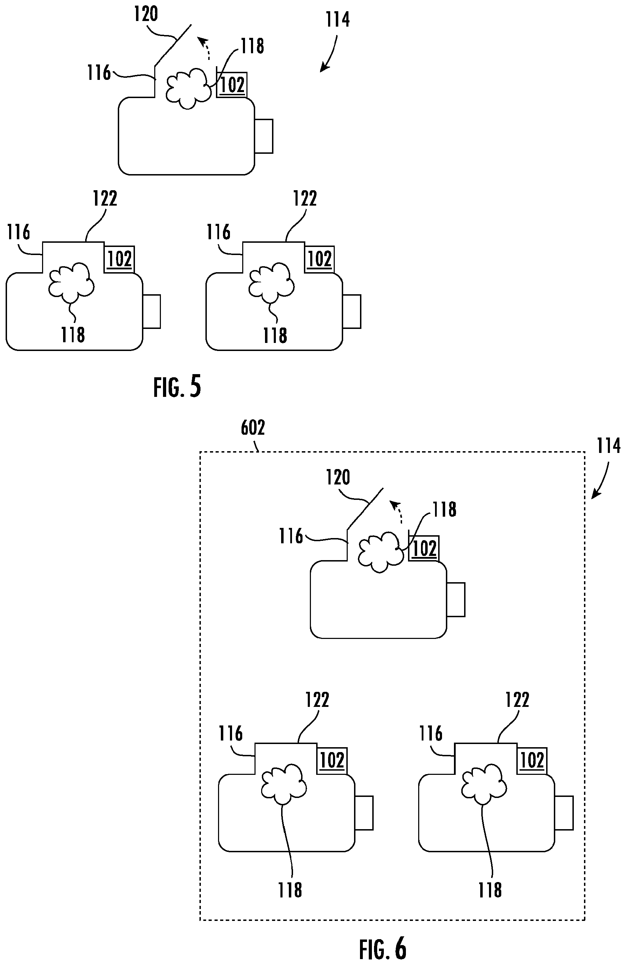

Although the sensor 102 and the one or more batteries 114 are depicted as separate components in FIG. 1, it would be understood by one skilled in the field to which this disclosure pertains that, in some embodiments, the sensor 102 and the one or more batteries 114 may be combined into a single component. For example, the sensor 102 may be integrated into one of the one or more batteries (e.g., attached to a surface of the one of the one or more batteries 114). Additionally or alternatively, although only one sensor 102 is depicted in FIG. 1, it would be understood by one skilled in the field to which this disclosure pertains that, in some embodiments, the system for detecting a vapor 100 may include more than one sensor 102. For example, as depicted in FIG. 5, the system for detecting a vapor 100 may include a sensor 102 for each of the one or more batteries 114. As another example, the system for detecting a vapor 100 may include a first sensor 102 for a portion of the one or more batteries 114 and a second sensor 102 for another portion of the one or more batteries 114.

In some embodiments, such as depicted in FIGS. 6-8, the one or more batteries 114 may be organized into one or more battery packs 602. In some embodiments, for example, each of the one or more batteries 114 in a battery pack 602 may be associated with a sensor 102. For example, a sensor 102 may be disposed proximate each of the one or more batteries 114 (e.g., a sensor 102 is attached to the surface of each of the one or more batteries 114). In some embodiments, for example, each of the one or more batteries 114 in a battery pack 602 may not be associated with a sensor 102. For example, a battery pack 602 having three batteries 114, may have two sensors 102 with each of the two sensors 102 associated with some or all of the batteries 114 is the battery pack 602 (e.g. each sensor 102 may be associated with batteries 114 proximate the sensor 102 in the battery pack 602). In some embodiments, the number of sensors 102 in the battery pack 602 may depend on the size of the battery pack 602 (e.g., the number of batteries 114 in the battery pack 602, the physical dimensions of the battery pack 602, etc.). In this regard, for example, the greater the size of the battery pack 602, the greater the number of sensors 102 in the battery pack 602.

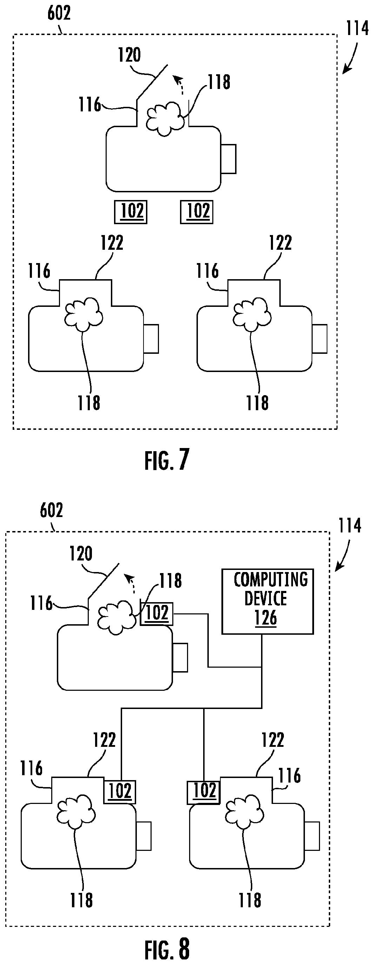

In some embodiments, such as depicted in FIG. 8, the one or more battery packs 602 may include a computing device, such as computing device 126. In this regard, for example, the computing device 126 may serve as battery pack management circuity for the battery pack 602. For example, the computing device 126 may be in communication with each of the sensors 102 in the battery pack 602. As described above, although the sensors 102 and the computing device 126 in battery pack 602 are depicted as separate components in FIG. 8, it would be understood by one skilled in the field to which this disclosure pertains that, in some embodiments, the sensors 102 and the computing device 126 may be combined into a single component. For example, the sensors 102 may integrated into the computing device 126 or the computing device 126 may be integrated into the sensor 102 (e.g., the sensors 102 may be integrated into the battery pack management circuity of the battery pack 602 or the battery pack management circuity of the battery pack 602 may be integrated into the sensors 102).

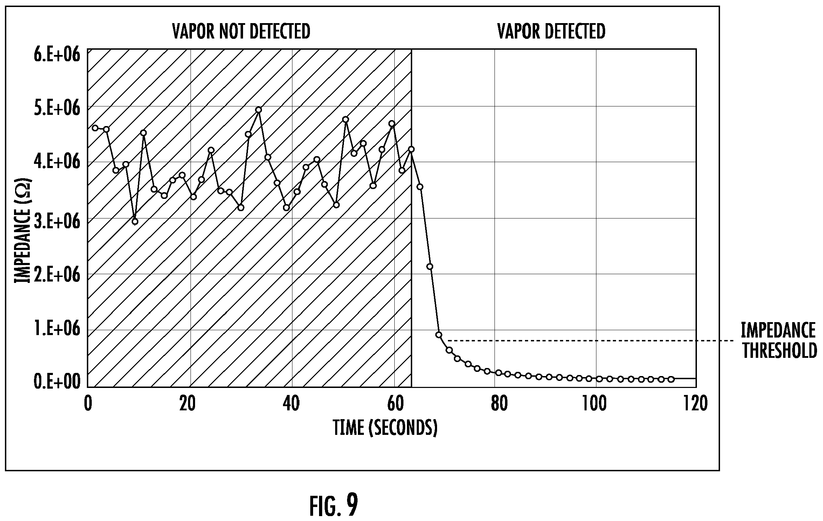

In some embodiments, the computing device 126 may be configured to measure an impedance of the polymer support 106 (e.g., using the pair of electrodes 110). In some embodiments, if the impedance of the polymer support 106 measured by the computing device 126 is below an impedance threshold, the computing device 126 may determine that a vapor 118 is detected. In this regard, as described above, absorbing at least some of a vapor 118 and, as a result, causing the polymer support 106 and/or the ionic salt 108 to solvate, causes a conductivity of the polymer support 106 to increase. In this regard, if the conductivity of the polymer support 106 increases, the impedance of the polymer support 106 will decrease. For example, as depicted in FIG. 9, when a vapor is not detected, the impedance of the polymer support 106 may be above the impedance threshold (e.g., the impedance of the polymer support 106 is approximately 4 MΩ). As another example, as depicted in FIG. 9, when a vapor is detected, the impedance of the polymer support 106 may be below the impedance threshold (e.g., the impedance of the polymer support 106 is less than 1MΩ). In this regard, for example, the system for detecting a vapor 100 may be capable of accurately detecting a vapor 118 (e.g., by measuring the impedance of the polymer support 106) with minimal power consumption by the sensor 102. For example, the average power consumption of the sensor 102 may be less than 50 microwatts (µW) (e.g., when the impedance is measured approximately once per second).

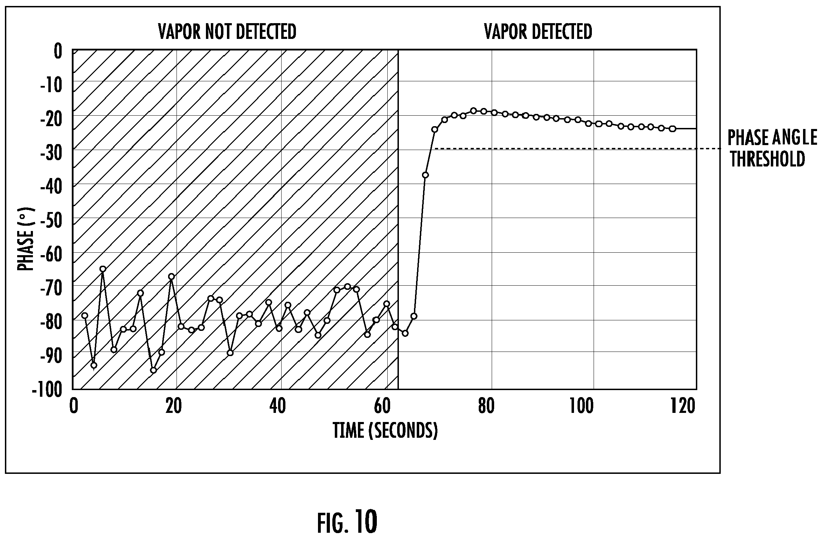

In some embodiments, the computing device 126 may be configured to measure a phase angle associated with the polymer support 106 (e.g., using the pair of electrodes 110 to determine the phase angle of the impedance of the polymer support 106). In some embodiments, if the phase angle associated with the polymer support 106 measured by the computing device 126 shifts such that the phase angle exceeds a phase angle threshold, the computing device 126 may determine that a vapor 118 is detected. In this regard, as described above, absorbing at least some of a vapor 118 and, as a result, causing the polymer support 106 and/or the ionic salt 108 to solvate, causes a conductivity of the polymer support 106 to increase. In this regard, if the conductivity of the polymer support 106 increases, the phase angle associated with the polymer support 106 may shift such that it exceeds a phase angle threshold. For example, as depicted in FIG. 10, when a vapor is not detected, the phase angle associated with the polymer support 106 does not exceed a phase angle threshold (e.g., the phase angle associated with the polymer support 106 is approximately - 80°). As another example, as depicted in FIG. 10, when a vapor is detected, the phase angle associated with the polymer support 106 may have shifted such that the phase angle exceeds the phase angle threshold (e.g., the phase angle associated with the polymer support 106 is approximately -20°). In this regard, for example, the system for detecting a vapor 100 may be capable of accurately detecting a vapor 118 (e.g., by measuring the phase angle associated with the polymer support 106) with minimal power consumption by the sensor 102. For example, the average power consumption of the sensor 102 may be less than 50 microwatts (µW) (e.g., when the phase angle is measured approximately once per second).

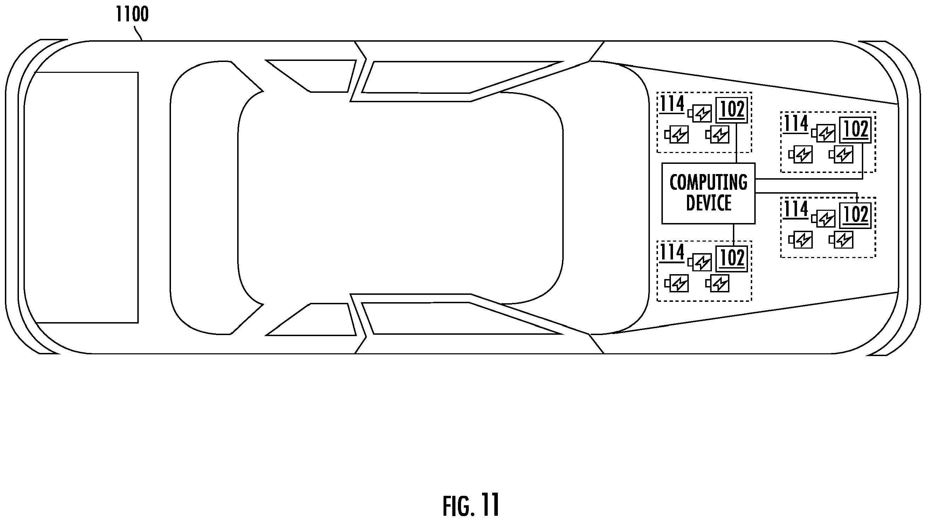

In some embodiments, such as depicted in FIG. 11, the system for detecting a vapor 100 may be implemented in an electric vehicle, such as electric vehicle 1100. In this regard, for example, electric vehicle 1100 may include one or more batteries 114, one or more sensors 102, and/or a computing device 126. In some embodiments, for example, the one or more batteries 114 in electric vehicle 1100 may be organized into one or more battery packs with each of the one or more battery packs being associated with one or more sensors 102. In some embodiments, the computing device 126 may serve as battery management circuity for the electric vehicle 1100. Although depicted as part of the electric vehicle 1100 in FIG. 11, it would be understood by one skilled in the field to which this disclosure pertains that, in some embodiments, the computing device 126 may be separate from the electric vehicle 1100 (e.g., the electric vehicle 1100 may include the sensor 102 and the one or more batteries 114). In this regard, for example, the computing device 126 may be a remote computing device with which the sensors 102 communicate with.

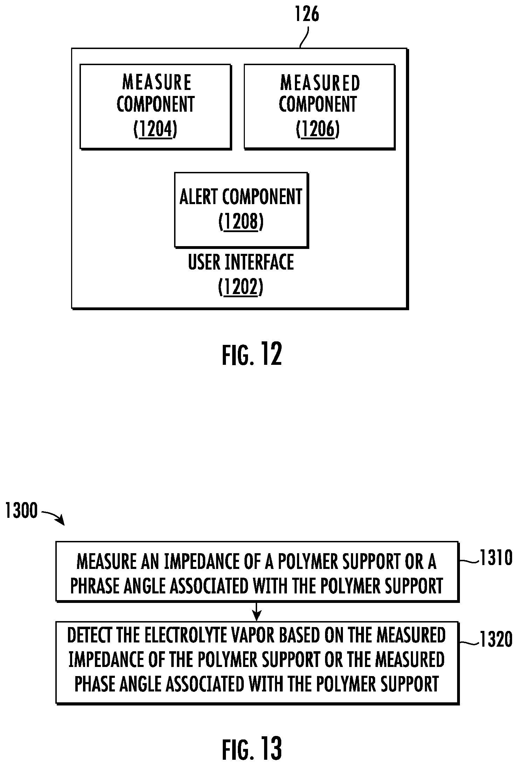

With reference to FIG. 12, in some embodiments, the computing device 126 may include a user interface 1202. In some embodiments, the user interface 1202 embodies a user interface configured to be rendered on a native application associated with the computing device 126 (e.g., the user interface 1202 is rendered on a native application associated with the electric vehicle 1100). In some embodiments, the user interface 1202 embodies a web interface accessible by a browser or other web application. In this regard, the user interface 1202 may be accessible by a browser or other web application associated with the computing device 126.

In some embodiments, the user interface 1202 may include a measure component 1204. The measure component 1204 may be configured to be used to measure the impedance of the polymer support 106 and/or the phase angle associated with the polymer support 106. For example, a user of the system for detecting a vapor 100 may use the measure component 1204 to measure the impedance of the polymer support 106 and/or the phase angle associated with the polymer support 106 to determine if the sensor 102 has detected a vapor 118 (e.g., the polymer support 106 has absorbed at least some of a vapor 118 and the conductivity of the polymer support 106 has increased). In this regard, the user of the system for detecting a vapor 100 may use the measure component 1204 to ascertain whether the impedance of the polymer support 106 has decreased below the impedance threshold and/or the phase angle associated with the polymer support 106 has shifted such that the phase angel exceeds the phase angle threshold. In some embodiments, the measure component 1204 may include text, symbols, graphs, and/or colors that enable a user to use the measure component 1204 to measure the impedance of the polymer support 106 and/or the phase angle associated with the polymer support 106.

In some embodiments, the user interface 1202 of the computing device 126 may include a measured component 1206. The measured component 1206 may be configured to display the measured impedance of the polymer support 106 and/or the measured phase angle associated with the polymer support 106. In some embodiments, the measured component 1206 may display the measured impedance of the polymer support 106 and/or the measured phase angle associated with the polymer support 106 after a user of the system for detecting a vapor 100 has selected the measure component 1204 to measure the impedance of the polymer support 106 and/or the measured phase angle associated with the polymer support 106. Additionally or alternatively, the system for detecting a vapor 100 may continuously and/or periodically (e.g., without a user selecting the measure component 1204 to cause the impedance of the polymer support 106 and/or the phase angle associated with the polymer support 106 to be measured) measure the impedance of the polymer support 106 and/or the phase angle associated with the polymer support 106. In this regard, for example, the measured component 1206 may display the most recent impedance measurement of the polymer support 106 and/or the most recent phase angle measurement associated with the polymer support 106, multiple impedance measurements and/or the phase angle measurements associated with the polymer support 106 made over a time period (e.g., to show a trend), and/or an impedance measurement that is below the impedance threshold and/or the a phase angle measurement that in which the phase angle associated with the polymer support has shifted such that the phase angle exceeds the phase angle threshold. In some embodiments, the measured component 1206 may include text, symbols, graphs, and/or colors that indicate the measured impedance of the polymer support 106 and/or the measured phase angle associated with the polymer support 106.

In some embodiments, the user interface 1202 of the computing device 126 may include an alert component 1208. In some embodiments, the alert component 1208 may indicate to a user of the system for detecting a vapor 100 that a vapor 118 has been detected. In this regard, the alert component 1208 may indicate to the user when the impedance of the polymer support 106 is below the impedance threshold and/or the phase angle associated with the polymer support 106 has shifted such that the phase angle exceeds the phase angle threshold (e.g., indicating that the conductivity of the polymer support 106 has increased and that one of the one or more batteries 114 may be in thermal runaway). As a result, in response to an indication on the alert component 1208, a user of the system for detecting a vapor 100 may be able to rapidly take corrective action to prevent a catastrophic failure of one or more of the one or more batteries 114. In some embodiments, the alert component 1208 may include text, symbols, graphs, and/or colors that indicate to a user of the system for detecting a vapor 100 that a vapor 118 has been detected (e.g., because the impedance of the polymer support 106 is below the impedance threshold and/or the phase angle associated with the polymer support 106 has shifted such that the phase angle exceeds the phase angle threshold). For example, the alert component 1208 may be a warning light in the electric vehicle 1100. As another example, the alert component 1208 may be a notification from a native application of the computing device 126.

Although the measure component 1204, the measured component 1206, and/or the alert component 1208 are depicted as separate components of the user interface 1202, it would be understood by one skilled in the field to which this disclosure pertains that, in some embodiments, the measure component 1204, the measured component 1206, and/or the alert component 1208 may be combined into a single component on the user interface 1202. For example, the measured component 1206 and the alert component 1208 may be combined into a single component on the user interface 1202.

In some embodiments, the computing device 126 may be configured to automatically take corrective action to prevent a catastrophic failure of one or more of the one or more batteries 114 when the computing device 126 determines that the impedance of the polymer support 106 is below the impedance threshold and/or the phase angle associated with the polymer support 106 has shifted such that the phase angle exceeds the phase angle threshold. For example, if the one or more batteries 114 are being charged when the computing device 126 determines that the impedance of the polymer support 106 is below the impedance threshold and/or the phase angle associated with the polymer support 106 has shifted such that the phase angle exceeds the phase angle threshold, the computing device 126 may be configured to automatically stop the charging of the batteries 114. As another example, if the one or more batteries 114 are supplying power (e.g., the electric vehicle 1100 is on) the computing device 126 determines that the impedance of the polymer support 106 is below the impedance threshold and/or the phase angle associated with the polymer support 106 has shifted such that the phase angle exceeds the phase angle threshold, the computing device 126 may be configured to cause the one or more batteries 114 to stop supplying power (e.g., shut down the electric vehicle 1100).

### Example Method of Detecting a Vapor

Referring now to FIG. 13, a flowchart providing an example method of detecting a vapor 1300 is illustrated. In this regard, FIG. 13 illustrates operations that may be performed by the system for detecting a vapor 100 and/or components of the system for detecting a vapor 100. For example, in some embodiments, the operations illustrated in FIG. 13 may, for example, be performed by, with the assistance of, and/or under the control of the computing device 126 (e.g., using processing circuitry 1402, memory 1404, processor 1406, user interface 1408, and/or communication interface 1410), the sensor 102, and/or the one or more batteries 114.

As shown in block 1310, the method for detecting a vapor 1300 may include measuring an impedance of a polymer support or a phase angle associated with the polymer support. As described above, in some embodiments, the polymer support may be disposed on a substrate such that the polymer support is in contact with a pair of electrodes disposed on the substrate. In some embodiments, the polymer support may include an ionic salt configured to absorb at least some of a vapor. In some embodiments, when the polymer support absorbs at least some of the vapor a conductivity of the polymer support may increase. In some embodiments, the polymer support absorbing at least some of the vapor, causes the polymer support and/or the ionic salt to solvate.

As described above, the polymer support may be comprised of one or more thermoplastics (e.g., a non-reactive thermoplastic). For example, the polymer support may comprise one or more of poly(ethyl methacrylate), poly(butyl methacrylate-co-methyl methacrylate), poly(methyl methacrylate-co-ethyl acrylate), poly(ethylene oxide) (PEO), poly(vinyl pyrrolidone) (PVP), poly(acrylonitrile) (PAN), poly(vinyl acetate) (PVAc), poly(vinyl butyral-co-vinyl alcohol-co-vinyl acetate), poly(ethylene-co-vinyl acetate), poly(1-vinylpyrrolidone-co-vinyl acetate), poly(methyl methacrylate) (e.g., PMMA), poly(vinylidene fluoride) (e.g., PVDF), poly(vinylidene fluoride-co-trifluoroethylene) (e.g., PVDF-TrFE), Poly(vinylidene fluoride-co-hexafluoropropylene) (e.g., PVDF-HEP), poly(dimethyldiallylammonium) bis(fluorosulfonyl)imide (e.g., PDDA FSI), or poly(dimethylpyrrolidinium) bis(trifluoromethylsulfonyl)imide (e.g., PDP TFSI). As described above, the ionic salt 108 may be comprised of one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide. As described above, the vapor may comprise one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

As described above, the vapor may be released from one or more batteries. In this regard, for example, a vent of one of the one or more batteries may be configured to move from the closed position to the open position to release a vapor from the battery and, in some embodiments, heat from the battery. In this regard, in some embodiments, as the temperature of one of the one or more batteries increases, a vapor may build up inside the battery. In some embodiments, once a certain amount of the vapor has built up inside one of the one or more batteries (e.g., when the temperature of the battery is above the temperature threshold), the vent may move from the closed position to the open position to release the vapor and reduce the pressure inside the battery. In this regard, the release of the vapor may indicate that the battery is not operating normally (e.g., the battery is overheating and going into thermal runaway).

As shown in block 1320, the method for detecting a vapor 1300 may include detecting the vapor based on the measured impedance of the polymer support or the measured phase angle associated with the polymer support. As described above, in some embodiments, if the impedance of the polymer support is below an impedance threshold, a vapor is detected. In this regard, as described above, absorbing at least some of a vapor and, as a result, causing the polymer support and/or the ionic salt to solvate, causes a conductivity of the polymer support to increase. In this regard, if the conductivity of the polymer support increases, the impedance of the polymer support will decrease. As described above, in some embodiments, if the phase angle associated with the polymer support 106 shifts such that the phase angle exceeds a phase angle threshold, a vapor 118 is detected. In this regard, as described above, absorbing at least some of a vapor 118 and, as a result, causing the polymer support 106 and/or the ionic salt 108 to solvate, causes a conductivity of the polymer support 106 to increase. In this regard, if the conductivity of the polymer support 106 increases, the phase angle associated with the polymer support 106 may shift such that the phase angle exceeds a phase angle threshold.

### Example Computer Processing Device

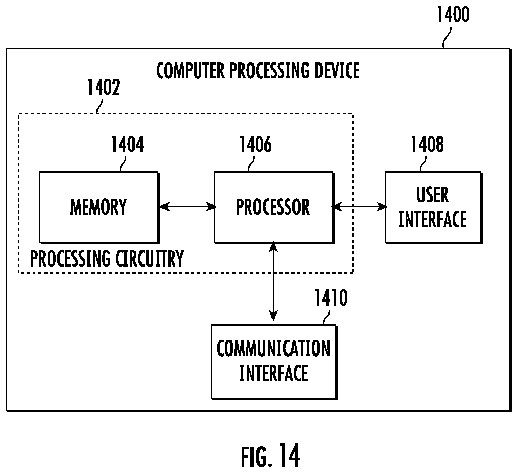

With reference to FIG. 14, a block diagram of an example computer processing device 1400 is illustrated in accordance with some example embodiments. In some embodiments, the computing device 126 (e.g., the battery pack management circuitry, the battery management circuitry, etc.) and/or other devices may be embodied as one or more computer processing devices, such as the computer processing device 1400 in FIG. 14. However, it should be noted that the components, devices, or elements illustrated in and described with respect to FIG. 14 below may not be mandatory and thus one or more may be omitted in certain embodiments. Additionally, some embodiments may include further or different components, devices or elements beyond those illustrated in and described with respect to FIG. 14.

The computer processing device 1400 may include or otherwise be in communication with processing circuitry 1402 that is configurable to perform actions in accordance with one or more embodiments disclosed herein. In this regard, the processing circuitry 1402 may be configured to perform and/or control performance of one or more functionalities of the computer processing device 1400 in accordance with various embodiments, and thus may provide means for performing functionalities of the computer processing device 1400 in accordance with various embodiments. The processing circuitry 1402 may be configured to perform data processing, application execution and/or other processing and management services according to one or more embodiments. In some embodiments, the computer processing device 1400 or a portion(s) or component(s) thereof, such as the processing circuitry 1402, may be embodied as or comprise a chip or chip set. In other words, the computer processing device 1400 or the processing circuitry 1402 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The computer processing device 1400 or the processing circuitry 1402 may therefore, in some cases, be configured to implement an embodiment of the disclosure on a single chip or as a single "system on a chip." As such, in some cases, a chip or chipset may constitute means for performing one or more operations for providing the functionalities described herein.

In some embodiments, the processing circuitry 1402 may include a processor 1406 and, in some embodiments, such as that illustrated in FIG. 14, may further include memory 1404. The processing circuitry 1402 may be in communication with or otherwise control a user interface 1408 and/or a communication interface 1410. As such, the processing circuitry 1402 may be embodied as a circuit chip (e.g., an integrated circuit chip) configured (e.g., with hardware, software or a combination of hardware and software) to perform operations described herein.

The processor 1406 may be embodied in a number of different ways. For example, the processor 1406 may be embodied as various processing means such as one or more of a microprocessor or other processing element, a coprocessor, a controller or various other computing or processing devices including integrated circuits such as, for example, an ASIC (application specific integrated circuit), an FPGA (field programmable gate array), or the like. Although illustrated as a single processor, it will be appreciated that the processor 1406 may comprise a plurality of processors. The plurality of processors may be in operative communication with each other and may be collectively configured to perform one or more functionalities of the computer processing device 1400 as described herein. In some embodiments, the processor 1406 may be configured to execute instructions stored in the memory 1404 or otherwise accessible to the processor 1406. As such, whether configured by hardware or by a combination of hardware and software, the processor 1406 may represent an entity (e.g., physically embodied in circuitry - in the form of processing circuitry 1402) capable of performing operations according to embodiments of the present disclosure while configured accordingly. Thus, for example, when the processor 1406 is embodied as an ASIC, FPGA or the like, the processor 1406 may be specifically configured hardware for conducting the operations described herein. Alternatively, as another example, when the processor 1406 is embodied as an executor of software instructions, the instructions may specifically configure the processor 1406 to perform one or more operations described herein.

In some embodiments, the memory 1404 may include one or more non-transitory memory devices such as, for example, volatile and/or non-volatile memory that may be either fixed or removable. In this regard, the memory 1404 may comprise a non-transitory computer-readable storage medium. It will be appreciated that while the memory 1404 is illustrated as a single memory, the memory 1404 may comprise a plurality of memories. The memory 1404 may be configured to store information, data, applications, instructions and/or the like for enabling the computer processing device 1400 to carry out various functions in accordance with one or more embodiments. For example, the memory 1404 may be configured to buffer input data for processing by the processor 1406. Additionally or alternatively, the memory 1404 may be configured to store instructions for execution by the processor 1406. As yet another alternative, the memory 1404 may include one or more databases that may store a variety of files, contents or data sets. Among the contents of the memory 1404, applications may be stored for execution by the processor 1406 in order to carry out the functionality associated with each respective application. In some cases, the memory 1404 may be in communication with one or more of the processor 1406, user interface 1408, and/or communication interface 1410 via a bus(es) for passing information among components of the computer processing device 1400.

The user interface 1408 may be in communication with the processing circuitry 1402 to receive an indication of a user input at the user interface 1408 and/or to provide an audible, visual, mechanical or other output to the user. As such, the user interface 1408 may include, for example, a keyboard, a mouse, a joystick, a display, a touch screen display, a microphone, a speaker, and/or other input/output mechanisms. As such, the user interface 1408 may, in some embodiments, provide means for a user to access and interact with the computing device 126 and/or the sensor 102.

The communication interface 1410 may include one or more interface mechanisms for enabling communication with other devices and/or networks. In some cases, the communication interface 1410 may be any means such as a device or circuitry embodied in either hardware, or a combination of hardware and software that is configured to receive and/or transmit data from/to a network and/or any other device or module in communication with the processing circuitry 1402. By way of example, the communication interface 1410 may be configured to enable the computing device 126 to communicate with the sensor 102 and/or other computing devices. Accordingly, the communication interface 1410 may, for example, include an antenna (or multiple antennas) and supporting hardware and/or software for enabling communications with a wireless communication network (e.g., a wireless local area network, cellular network, global positing system network, and/or the like) and/or a communication modem or other hardware/software for supporting communication via cable, digital subscriber line (DSL), universal serial bus (USB), Ethernet or other methods.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

While various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

Use of broader terms such as "comprises," "includes," and "having" should be understood to provide support for narrower terms such as "consisting of," "consisting essentially of," and "comprised substantially of" Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

## Claims

1. A sensor for detecting a vapor, the sensor comprising:
a substrate;
a pair of electrodes disposed on the substrate; and
a polymer support disposed on the substrate such that the polymer support is in contact with the pair of electrodes, wherein the polymer support comprises an ionic salt, wherein the polymer support is configured to absorb at least some of the vapor, wherein, when the polymer support absorbs at least some of the vapor, a conductivity of the polymer support increases.

2. The sensor of claim 1, wherein the polymer support absorbing at least some of the vapor causes the ionic salt to solvate.

3. The sensor of claim 1, wherein the ionic salt comprises one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide.

4. The sensor of claim 1, wherein the polymer support comprises one or more of poly(ethyl methacrylate), poly(butyl methacrylate-co-methyl methacrylate), poly(methyl methacrylate-co-ethyl acrylate), poly(ethylene oxide), poly(vinyl pyrrolidone), poly(acrylonitrile), poly(vinyl acetate), poly(vinyl butyral-co-vinyl alcohol-co-vinyl acetate), poly(ethylene-co-vinyl acetate), poly(1-vinylpyrrolidone-co-vinyl acetate), poly(methyl methacrylate), poly(vinylidene fluoride), poly(vinylidene fluoride-co-trifluoroethylene), poly(vinylidene fluoride-co-hexafluoropropylene), poly(dimethyldiallylammonium) bis(fluorosulfonyl)imide, or poly(dimethylpyrrolidinium) bis(trifluoromethylsulfonyl)imide.

5. The sensor of claim 1, wherein the vapor comprises one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

6. A system for detecting a vapor, the system comprising:
a battery;
a sensor disposed proximate the battery, wherein the sensor comprises:
a substrate;
a pair of electrodes disposed on the substrate; and
a polymer support disposed on the substrate such that the polymer support is in contact with the pair of electrodes, wherein the polymer support comprises an ionic salt, wherein the polymer support is configured to absorb at least some of the vapor, wherein, when the polymer support absorbs at least some of the vapor, a conductivity of the polymer support increases.

7. The system of claim 6, wherein the polymer support absorbing at least some of the vapor causes the ionic salt to solvate.

8. The system of claim 6, wherein the ionic salt comprises one or more of tetrabutylammonium tetrafluoroborate, tetraethylammonium tetrafluoroborate, tetramethylammonium tetrafluoroborate, lithium tetrafluoroborate, silver tetrafluoroborate, tetramethylammonium hexafluorophosphate, tetraethylammonium hexafluorophosphate, tetrabutylammonium hexafluorophosphate, tetrabutylammonium chloride, tetraethylammonium chloride, tetramethylammonium chloride, tetramethylammonium bis(trifluoromethylsulfonyl)imide, tetrabutylammonium bis(trifluoromethylsulfonyl)imide, tributylmethylammonium bis(trifluoromethylsulfonyl)imide, tetraethylammonium bis(trifluoromethylsulfonyl)imide, tetrabutylammonium triflate, tributylmethylammonium triflate, 1-ethyl-3-methylimidazolium bis(trifluoromethylsulfonyl) imide, or tri ethyl sulfonium bis(trifluoromethyl sulfonyl) imide.

9. The system of claim 6, wherein the polymer support comprises one or more of poly(ethyl methacrylate), poly(butyl methacrylate-co-methyl methacrylate), poly(methyl methacrylate-co-ethyl acrylate), poly(ethylene oxide), poly(vinyl pyrrolidone), poly(acrylonitrile), poly(vinyl acetate), poly(vinyl butyral-co-vinyl alcohol-co-vinyl acetate), poly(ethylene-co-vinyl acetate), poly(1-vinylpyrrolidone-co-vinyl acetate), poly(methyl methacrylate), poly(vinylidene fluoride), poly(vinylidene fluoride-co-trifluoroethylene), poly(vinylidene fluoride-co-hexafluoropropylene), poly(dimethyldiallylammonium) bis(fluorosulfonyl)imide, or poly(dimethylpyrrolidinium) bis(trifluoromethylsulfonyl)imide.

10. The system of claim 6, wherein the vapor comprises one or more of propylene carbonate (PC), ethylene carbonate (EC), diethyl carbonate (DEC), dimethyl carbonate (DMC), ethyl methyl carbonate (EMC), dimethoxyethane (DME), or gamma-butyrolactone (GBL).

11. The system of claim 6, wherein the battery releases the vapor when a temperature of the battery is greater than a temperature threshold.

12. The system of claim 6 further comprising:
a computing device in communication with the sensor and configured to measure an impedance of the polymer support or a phase angle associated with the polymer support.

13. A method of detecting a vapor comprising:
measuring an impedance of a polymer support, wherein the polymer support is disposed on a substrate such that the polymer support is in contact with a pair of electrodes disposed on the substrate, wherein the polymer support comprises an ionic salt, wherein the polymer support is configured to absorb at least some of the vapor, wherein, when the polymer support absorbs at least some of the vapor, a conductivity of the polymer support increases; and
detecting the vapor based on the measured impedance of the polymer support.

14. The method of claim 13, wherein the vapor is detected when the impedance of the polymer support is below an impedance threshold.

15. The method of claim 13, further comprising:
measuring a phase angle associated with the polymer support.
